(19) Europäisches Patentamt
European Patent Office
Office européen des brevets

(11) Veröffentlichungsnummer: **0 001 780**
**A1**

(12) # EUROPÄISCHE PATENTANMELDUNG

(21) Anmeldenummer: **78101154.9**

(22) Anmeldetag: **14.10.78**

(51) Int. Cl.[2]: **C 07 C 68/08**
C 07 C 69/96

(30) Priorität: **07.11.77 DE 2749756**

(43) Veröffentlichungstag der Anmeldung:
**16.05.79 Patentblatt 79/10**

(84) Benannte Vertragsstaaten:
**DE FR GB**

(71) Anmelder: **BASF Aktiengesellschaft**
**Carl-Bosch-Strasse 38**
**D-6700 Ludwigshafen(DE)**

(72) Erfinder: **Fischer, Karl, Dr.**
**Luginsland 22**
**D-6520 Worms(DE)**

(72) Erfinder: **Kaibel, Gerd**
**Wormser Strasse 78**
**D-6840 Lampertheim(DE)**

(72) Erfinder: **Himmele, Walter, Dr.**
**Eichenweg 14**
**D-6909 Walldorf(DE)**

(72) Erfinder: **Irnich, Rudolf, Dr.**
**In den Hahndornen 2**
**D-6719 Bobenheim(DE)**

(72) Erfinder: **Schneider, Kurt, Dr.**
**Auf dem Koeppel**
**D-6702 Bad Duerkheim 1(DE)**

(54) **Verfahren zur Reindarstellung von Dimethylcarbonat.**

(57) Reindarstellung von Dimethylcarbonat aus seinen methanolischen Lösungen, indem man das Methanol aus diesen Lösungen durch Azeotropdestillation mit einem $C_5$-$C_8$-Paraffin oder-Cycloparaffin entfernt.

EP 0 001 780 A1

# Verfahren zur Reindarstellung von Dimethylcarbonat

Die vorliegende Erfindung betrifft ein neues Verfahren zur Reindarstellung von Dimethylcarbonat aus seinen methanolischen Lösungen.

Dimethylcarbonat wird üblicherweise (vgl. z.B. US-PS 3 803 201) durch Umesterung von Äthylencarbonat mit Methanol hergestellt:

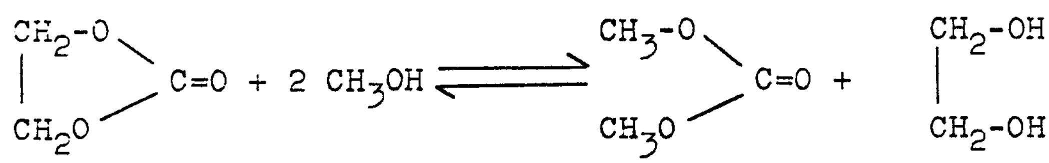

Da es sich hierbei um eine Gleichgewichtsreaktion handelt, muß man das Dimethylcarbonat laufend aus dem Reaktionsgemisch abdestillieren. Dies ist jedoch mit dem Nachteil verbunden, daß Dimethylcarbonat und Methanol ein Azeotrop bilden, so daß man, je nach dem herrschenden Druck, bis zu 30-gewichtsprozentige methanolische Lösungen des gewünschten Verfahrensproduktes erhält. Aus der oben erwähnten Patentschrift ist es bekannt, das Dimethylcarbonat zu seiner Reindarstellung aus diesen Lösungen bei etwa (-70)$^{o}$C auszukristallisieren, eine aufwendige Methode, die für technische Verfahren so gut wie unbrauchbar ist.

Mi/Fe

Mi

Auch die Druckdestillation solcher Gemische bei 10 bar nach der Arbeitsweise der DE-OS 26 07 003 stellt keine befriedigende Lösung des Trennungsproblems dar, weil sie einen großen apparativen und energetischen Aufwand erfordert und die Zusammensetzung des Azeotrops lediglich zugunsten des Dimethylcarbonats verschiebt.

Schließlich ist es aus der DE-OS 24 50 856 bekannt, Gemische aus Methanol, Wasser und Dimethylcarbonat der Extraktivdestillation mit Wasser zu unterwerfen. Abgesehen von der hierbei auftretenden Hydrolysegefahr ist auch dieses Verfahren unvorteilhaft, weil das im Überschuß vorhandene Methanol in die wäßrige Phase geht und hiervon nur unter erheblichem Wärmeverbrauch wieder abgetrennt werden kann.

Der Erfindung lag daher die Aufgabe zugrunde, Dimethylcarbonat aus seinen methanolischen Lösungen auf wirtschaftlichere Weise zu gewinnen als bisher.

Es wurde gefunden, daß man Dimethylcarbonat aus seinen methanolischen Lösungen auf wirtschaftliche Weise gewinnen kann, indem man das Methanol aus diesen Lösungen durch Azeotropdestillation mit einem $C_5$-$C_8$-Paraffin oder -Cycloparaffin entfernt.

Es wurde weiterhin gefunden, daß sich Cyclopentan, Cyclohexan und n-Hexan für den erfindungsgemäßen Zweck am besten eignen, weil diese Schleppmittel praktisch keine ternären, Dimethylcarbonat enthaltenden Azeotrope bilden.

Der besondere Vorteil des erfindungsgemäßen Verfahrens liegt darin, daß sich die azeotropen Gemische nach ihrer Kondensation in eine Paraffin- bzw. Cycloparaffinphase und eine Methanolphase trennen. Die Methanolphase, welche je nach Art des Schleppmittels noch etwas Dimethylcarbonat

enthalten kann, wird in die Umesterungsstufe zurückgeführt, während die paraffinische bzw. cycloparaffinische Phase bei der Azeotropdestillation der methanolischen Dimethylcarbonatlösung unmittelbar im Kreis geführt werden kann. Man benötigt daher nur soviel des Schleppmittels wie zur Aufrechterhaltung einer zweckmäßigen Konzentration von 1 bis 50 Mol%, bezogen auf die Menge des Methanols in der methanolischen Dimethylcarbonatlösung, erforderlich ist.

Die folgende Tabelle gibt eine Übersicht über die Eigenschaften einiger Azeotrope mit den gebräuchlichsten Paraffinen bzw. Cycloparaffinen als Schleppmitteln.

| Schleppmittel S | Zusammensetzung des Azeotrops in Gew.% S | Methanol | DMC+) | Siedepunkt++) des Azeotrops °C |
|---|---|---|---|---|
| n-Hexan | 67 | 33 | 0 | 51 |
| n-Heptan | 48 | 45 | 7 | 60 |
| n-Octan | 21 | 64 | 15 | 64 |
| Cyclopentan | 81 | 19 | 0 | 40 |
| Cyclohexan | 62 | 37 | 1 | 55 |

+) Dimethylcarbonat
++) bei Normaldruck

Aus diesen Daten, die für weitere definitionsgemäße Schleppmittel oder auch von deren Gemischen - insbesondere technischen Gemischen, wie Benzinfraktionen - unschwer zu ermitteln sind, ergeben sich die Arbeitsbedingungen für das erfindungsgemäße Verfahren von selbst, so daß nähere Ausführungen hierüber entbehrlich sind.

Das bei der Azeotropdestillation jeweils zurückbleibende Dimethylcarbonat liegt in aller Regel in einer hinreichend reinen Form vor, so daß es keines weiteren Reinigungsschrittes mehr bedarf. Dimethylcarbonat ist bekanntermaßen ein wichtiges Zwischenprodukt für die Herstellung von Pflanzenschutzmitteln, Pharmaka und Farbstoffen.

Beispiel

76 kg einer Lösung aus 70 Gew.% Methanol und 30 Gew.% Dimethylcarbonat, wie sie als Azeotrop bei der Herstellung von Dimethylcarbonat aus Äthylencarbonat und Methanol anfiel, wurden zusammen mit 30 kg n-Hexan der Azeotropdestillation unterworfen (Siedepunkt des Azeotrops 51°C). Nach vollständiger Entfernung des Methanols und der Abnahme eines Zwischenlaufs von 4 kg (Siedepunkt 52°C - 90°C), der hauptsächlich aus n-Hexan und Dimethylcarbonat bestand, blieb das Dimethylcarbonat in einer Ausbeute von 78 % in reiner Form zurück. Das restliche Dimethylcarbonat ging in die methanolische Phase des Azeotrops, welche nach ihrer Abtrennung von der n-Hexan-Phase in die Umesterungsstufe zurückgeführt wurde.

Patentansprüche

1. Verfahren zur Reindarstellung von Dimethylcarbonat aus seinen methanolischen Lösungen, dadurch gekennzeichnet, daß man das Methanol aus diesen Lösungen durch Azeotropdestillation mit einem $C_5$-$C_8$-Paraffin oder -Cycloparaffin entfernt.

2. Verfahren nach Anspruch 1, dadurch gekennzeichnet, daß man als $C_5$-$C_8$-Paraffin bzw. -Cycloparaffin n-Hexan, Cyclohexan oder Cyclopentan verwendet.

0001780

Europäisches Patentamt

# EUROPÄISCHER RECHERCHENBERICHT

Nummer der Anmeldung

EP 78 101 154.9

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | Chemical Abstracts Band 84, Nr. 19 1976,<br>Columbus, Ohio, USA<br>I. TAKASHI et al "Separation of naphthenic hydrocarbons-alcohol mixtures by distillation"<br>Seite 125, Spalte 1, Abstract Nr. 107 737g<br>& JP - A - 75 112 309<br>-- | 1 | C 07 C 68/08<br>C 07 C 69/96 |
| | | | RECHERCHIERTE SACHGEBIETE (Int. Cl.²) |
| A | DE - A - 2 528 412 (SNAMPROGETTI)<br>* Beispiel 9 *<br>---- | 1,2 | C 07 C 68/08<br>C 07 C 69/96 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 25-01-1979 | KNAACK |